# NEW EUROPEAN PATENT SPECIFICATION

(11) **EP 1 362 568 B2**
(45) Date of publication and mention of the opposition decision: **21.09.2011**
(45) Mention of the grant of the patent: 28.06.2006
(21) Application number: 03251527.2
(22) Date of filing: 13.03.2003
(51) Int. Cl.: A61F 13/15

(54) **Absorbent article**
Absorbierender Artikel
Article absorbant

(30) Priority: 26.03.2002 JP 2002085004; 19.11.2002 JP 2002335126
(43) Date of publication of application: 19.11.2003
(73) Proprietor: UNI-CHARM CORPORATION, Shikokuchuo-shi, Ehime-ken (JP)
(72) Inventor: Kudo, Jun, Technical Center, Mitoyo-gun, Kagawa-ken 769-1602 (JP); Suekane, Makoto, c/o Technical Center, Mitoyo-gun, Kagawa-ken 769-1602 (JP); Ito, Yukihiro, Technical Center, Mitoyo-gun, Kagawa-ken 769-1602 (JP); Sato, Junko, c/o Technical Center, Mitoyo-gun, Kagawa-ken 769-1602 (JP)
(74) Representative: Eke, Philippa Dianne

(56) References cited:
- EP-A- 1 101 468
- EP-A1- 0 895 766
- EP-B1- 0 072 948
- WO-A1-00/35503
- WO-A1-93/09744
- WO-A1-98/27908
- WO-A1-99/58092
- US-A- 3 929 135
- US-A- 4 518 451
- US-A- 4 634 440
- US-A- 5 308 346
- US-A- 5 891 118
- US-A- 6 117 523
- US-B1- 6 204 210
- DATABASE WPI Section Ch, Week 199851 Derwent Publications Ltd., London, GB; Class A96, AN 1998-602141 XP002301544 -& JP 10 272152 A (UNI-CHARM KK) 13 October 1998 (1998-10-13)

## Description

### BACKGROUND OF THE INVENTION

### Field of the Invention

The present invention relates to an absorbent article, more particularly, relates to a thin absorbent article, such as panty liner, that is suitable for absorbing a relatively small amount of discharged body fluid.

### Description of the Related Art

Although high absorbency is not required, panty liners have to be able to absorb a relatively small amount of body fluids such as vaginal discharge. In addition, they are to be used in daily life, it is preferred that they do not give an unpleasant feeling during wear. It is also preferred that they have a beautiful design for making them comfortable for wearers. Therefore, as disclosed in Japanese Unexamined Patent Publication Nos. 2001-145669 and 2001-231816, conventional panty liners are made narrow and small enough to fit on a crotch portion of a short panty and made thin as a whole by using a thin absorbent body, wherein a top material layer is often formed of relatively bulky, porous nonwoven fabric that has a soft feeling and an excellent liquid-permeability.

However, since the conventional panty liners are made small to have a relatively narrow absorbent region, they are sometimes insufficient in absorbency even for absorbing a relatively small amount of body fluid.

In order to solve this problem, absorbency can be increased by making the absorbent body thick. In this case, however, if the entire thickness of the liner is increased, an unpleasant feeling may be given during wear. In some conventional panty liners, therefore, a relatively thick absorbent body is disposed along a longitudinally extending centerline of the liner so as to increase the absorbency without giving an unpleasant feeling during wear. In such conventional panty liners, however, since the absorbent body is disposed only in a central portion of the liner, body fluid flowing in the top material layer may possibly ooze out at laterally opposed side portions of the liner, particularly at a location near a laterally extending centerline of the liner.

In the panty liner disclosed in Japanese Unexamined Patent Publication No. 2001-145669, moreover, holes for passage of liquid are formed in the top material layer so as to improve the liquid-permeability of the top material layer. However, since these holes are distributed over the entire surface of the top material layer, body fluid easily passes through the top material layer in the side regions outside the absorbent body. Therefore, body fluid easily leaks laterally. In addition, since the holes pass through only the top material layer, liquid having passed through the holes easily flows laterally on the surface of the absorbent body between the top material layer and the absorbent body, which may also cause lateral leakage.

### SUMMARY OF THE INVENTION

The present invention has been worked out in view of the shortcoming in the prior art set forth above. It is therefore an object of the present invention to provide an absorbent article that is effective in preventing leakage of body fluids toward laterally opposed edges of the absorbent article.

According to the present invention, there is provided an absorbent article comprising:
a liquid-permeable top material layer;
a liquid-impermeable back material layer; and
an absorbent body that is narrower than the top material layer and the back material layer and disposed between the top material layer and the back material layer such that longitudinally extending side edges of the absorbent body are individually inwardly spaced apart from corresponding side edges of the top material layer and the back material layer, wherein
along the individual side edges of the absorbent body, the top material layer only is heat-embossed to have compressed portions, wherein the compressed portions are arranged in a longitudinally spaced relation to each other so that the compressed portions are formed in a substantially continuous, longitudinally extending pattern with regions having no compressed portions left between longitudinally adjacent compressed portions so that at least 80% of each side edge overlaps with the compressed portions, when the top material layer is viewed from a direction perpendicular to each side edge of the absorbent body in a plane substantially flush with the top material layer,
wherein the compressed portions all comprise linear compressed portions, which each extend to surround an uncompressed portion without interruption, and the linear compressed portions form a pattern repeated along the individual side edges of the absorbent body, the compressed portions being provided in regions riding on the side edges of the absorbent body to leave a non embossed region between the regions having the compressed portions.

US 6204210 discloses all the features of claim 1 except the feature of longitudinally adjacent compressed portions having regions left between then having no compressed portions and the feature of the compressed portions being provided in regions riding on the side edges of the absorbent body.

In this absorbent article, since body fluid diffusing laterally in the top material layer can be effectively blocked by the compressed portions of the substantially continuous, longitudinally extending pattern, the body fluid can be certainly absorbed by the absorbent body. Thus, the body fluid can be effectively prevented from leaking from laterally opposed side edges of the absorbent article.

Since the compressed portions overlap with side portions of the absorbent body, body fluid can be certainly prevented from diffusing beyond the side edges of the absorbent body by the compressed portions. Therefore, the body fluid can be certainly absorbed by the underlying absorbent body. In addition, since the regions having the compressed portions not only overlap with the absorbent body but also extend outwardly beyond the side edges of the absorbent body, the nonembossed region of the top material layer through which liquid can easily pass can be made large.

The linear compressed portions are effective in blocking body fluid. Since each linear compressed portion extends to surround an uncompressed portion without interruption, the uncompressed portion surrounded by the linear compressed portion can further enhance the effect of blocking body fluid.

The linear compressed portions may form a pattern of leaves so as to provide a good appearance.

Preferably, liquid passage holes extending through the top material layer into the absorbent body are formed in a region where the absorbent body is present. More preferably, the liquid passage holes are distributed within a nonembossed region left between regions having the compressed portions so that all the liquid passage holes are spaced apart from the compressed portions. The liquid passage holes thus formed facilitate absorption of body fluid into the absorbent body. Particularly when the liquid passage holes are distributed so as not to overlap with the compressed portions, body fluid given to the nonembossed region can be promptly absorbed by the absorbent body through the liquid passage holes, while lateral diffusion of the body fluid is certainly prevented by the compressed portions. Therefore, lateral leakage of body fluid can be prevented more effectively.

### BRIEF DESCRIPTION OF THE DRAWINGS

The present invention will be understood more fully from the detailed description given hereinafter and from the accompanying drawings of the preferred embodiments of the present invention, which, however, should not be taken to be limitative to the invention, but are for explanation and understanding only.

In the drawings:
Fig. 1A is a schematic sectional view taken along a laterally extending centerline of an absorbent article according to one embodiment of the present invention, and Fig. 1B is a partially enlarged view of Fig. 1A;
Fig. 2A is a top plan view of the absorbent article of Fig. 1A, wherein a top material layer is heat-embossed in a pattern of leaves, and Fig. 2B is a partially enlarged view of Fig. 2A.

### DESCRIPTION OF THE PREFERRED EMBODIMENT

The present invention will be discussed hereinafter in detail in terms of the preferred embodiments according to the present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structures are not shown in detail in order to avoid unnecessary obscurity of the present invention.

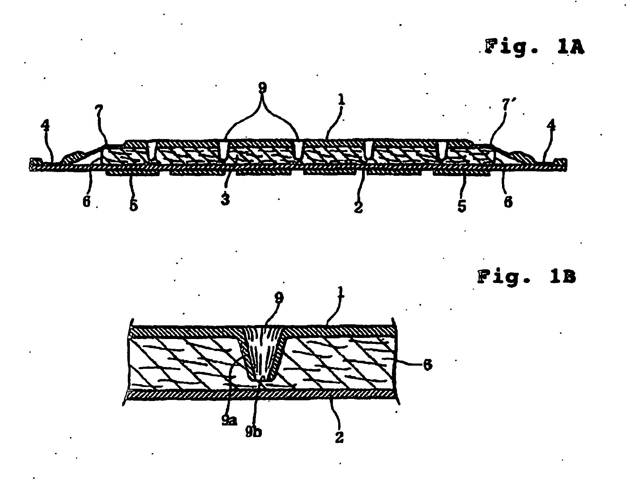

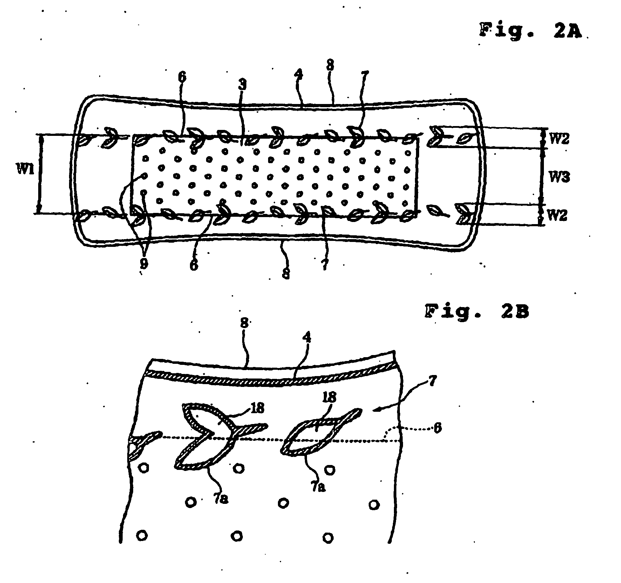

Fig. 1A is a schematic sectional view taken along a laterally extending centerline of an absorbent article according to one embodiment of the present invention; Fig. 1B is a partially enlarged view of Fig. 1A; Fig. 2A is a top plan view of the absorbent article of Fig. 1A; and Fig. 2B is a partially enlarged view of Fig. 2A.

As used herein, the term " absorbent article" refers to a thin absorbent article, such as panty liner, that is suitable for absorbing a relatively small amount of body fluids such as vaginal discharge, menstrual blood and the like. This absorbent article is basically constructed to include a liquid-permeable top material layer 1, a liquid-impermeable back material layer 2, and an absorbent body 3 that is narrower than and disposed between the top material layer 1 and the back material layer 2. Along the periphery of the absorbent article, the liquid-permeable top material layer 1 and the liquid-impermeable back material layer 2 are joined together by joining means such as heat-sealing, thereby forming a seal 4 for leakage prevention. On the external surface of the liquid-impermeable back material layer 2, provided is a pressure sensitive adhesive layer 5 for securing the absorbent article such as panty liner on a crotch portion of an undergarment such as short panty.

### (Top Material Layer)

In the embodiment shown, soft, relatively bulky, porous, low-density liquid-permeable nonwoven fabric such as through-air bonded nonwoven fabric is used for the liquid-permeable top material layer 1. In order to improve the feel and bulk, two or more sheets of the relatively bulky porous nonwoven fabric may be laminated.

For the top material layer 1, it is preferred that the nonwoven fabric has a basis weight of 20 to 60 g/m² (in the case where two or more sheets of the nonwoven fabric are laminated, the total basis weight is 20 to 60 g/m²), a density of 0.12 g/cm³ or less, and a SMD (surface roughness) of 4.5 µm or less when measured using a Surface Tester (manufactured by KES Kato Tech Co., Ltd.). SMD means the mean deviation of surface roughness and has the same concept as the centerline average height. That is, SMD is a value that is obtained by integrating thickness differences between the average centerline of the surface roughness and the surface in the range of a distance X and dividing the integrated value by the distance X. According to the Surface Tester, the measurement is performed such that a contact formed by bending a piano wire having a diameter of 0.5 mm is brought into contact with the material surface under a force of 98 mN, wherein the distance X is 2 cm.

For the top material layer 1, preferably used is through-air bonded nonwoven fabric, because it has higher bulk than other kinds of nonwoven fabric (such as spunbonded, spunlaced and the like) and can be provided with a large number of porosities. In case where two sheets of through-air bonded nonwoven fabric are laminated for use in the top material layer 1, for example, the individual sheet of the through-air bonded nonwoven fabric may have a basis weight of 25 g/m² and comprise polyethylene/polyethylene terephthalate (containing 1.0% of titanium oxide) sheath/core bicomponent thermoplastic fibers (2 denier × 44 mm). In addition, the individual sheet of the through-air bonded nonwoven fabric is preferably treated with hot air for one minute at a temperature of 115 °C to restore the bulk from a thickness of 0.5 mm to a thickness of 1.0 mm. Since the sheath/core bicomponent fibers forming the through-air bonded nonwoven fabric contain titanium oxide in the core component, surface smoothness and drape are improved to provide an excellent texture, as compared with fibers not containing titanium oxide.

In case where only one sheet of nonwoven fabric is used for the top material layer 1, the top material layer 1 tends to be stiff because the nonwoven fabric need be of a large basis weight for increasing the bulk. In case where three or more sheets of nonwoven fabric are laminated, on the other hand, stiffness is increased by attachment means such as hot-melt adhesive for joining the sheets together. Accordingly, the top material layer 1 is preferably formed from a laminate of two sheets of through-air bonded nonwoven fabric. At this time, it is more preferred that the attachment means for joining the two sheets of through-air bonded nonwoven fabric together is reduced as much as possible. However, it is, of course, possible to use only one sheet or a laminate of three or more sheets as long as the physical properties are suitable for use as the top material layer.

The most important characteristic for obtaining the effects of the present invention is that the liquid-permeable top material layer 1 is embossed to have compressed portions 7. These compressed portions 7 are formed in a predetermined pattern by heat-embossing the top material layer 1 at two regions that extend along longitudinally extending side edges 6 of the absorbent body 3. The compressed portions 7 are provided in regions that ride on the side edges 6 of the absorbent body 3 (i.e., regions that range from positions above side portions of the absorbent body 3 to positions outside the absorbent body 3).

In this embodiment, as shown in Figs. 2A and 2B, the compressed portions 7 are arranged in longitudinally spaced relation to each other so that the compressed portions 7 are formed in a substantially continuous, longitudinally extending pattern. The term " substantially continuous, longitudinally extending pattern" as used herein means that when the top material layer 1 is viewed from a direction perpendicular to each side edge 6 of the absorbent body 3 in a plane substantially flush with the top material layer 1, at least 80% (preferably at least 90%, more preferably 100%) of each side edge 6 overlaps with compressed portions 7 that are arranged along the side edge 6.

In the embodiment shown in Figs. 2A and 2B, linear compressed portions 7a each surrounding an uncompressed portion 18 without interruption form a pattern of leaves. In the uncompressed portion 18, the top material layer 1 is not compressed at all or the top material layer 1 is substantially compressed at the time of forming the linear compressed portions 7a but with a compressibility sufficiently smaller than the linear compressed portions 7a.

Here, the pattern formed by the linear compressed portions 7a should not be limited to a pattern of leaves but may be a pattern of circles, stars, flowers or the like.

When heat-embossed, the nonwoven fabric is consolidated or melted into a film at the compressed portions 7. Accordingly, body fluid diffusing laterally in the top material layer 1 can be blocked by the compressed portions 7 to prevent lateral leakage or exudation. The body fluid thus blocked flows toward the underlying absorbent body 3 and is then absorbed by the absorbent body 3. Even in the case where the top material layer 1 is formed from two or more sheets of nonwoven fabric or a single sheet of thick nonwoven fabric, the top material layer 1 is preferably melted into a film by heat-embossing at the compressed portions 7 over the entire thickness thereof so as to certainly prevent lateral leakage (exudation).

In the embodiment of Figs. 2A and 2B, even though the compressed portions 7 are arranged in longitudinally spaced relation to each other, most of body fluid diffusing laterally in the top material layer 1 can be blocked by the compressed portions 7 near the side edges 6 of the absorbent body 3 to flow toward the absorbent body 3. Here, since a panty liner or the like is intended to absorb a small amount of body fluid, lateral leakage or exudation can be prevented almost perfectly when spaces (regions having no compressed portions) are left between longitudinally adjacent compressed portions 7.

Since the compressed portions 7 are arranged along the side edges 6 of the absorbent body 3, they do not prevent migration of body fluid from the top material layer 1 to the absorbent body 3, so that almost the entire surface of the absorbent body 3 can be fully exploited for absorption of body fluid.

Here, the compressed portions 7 are formed by heat-embossing only the top material layer 1. Accordingly, the stiffness of the entire absorbent article will not be greatly influenced even if the individual compressed portions are formed to have a large line thickness, the individual circles, leaves or the like are entirely consolidated by heat-embossing without leaving the uncompressed portions inside of them or the individual compressed portions are formed to have a large area. However, the compressed portions 7 should be formed so as not to damage the soft texture of the top material layer 1.

The line thickness of the linear compressed portions 7a is preferably at most 1.0 mm, more preferably at most 0.5 mm. With such line thickness, even if a large number of linear compressed portions are present, the soft texture of the top material layer 1 will not be damaged as well as the stiffness of the entire absorbent article will not be greatly influenced. That is, it is preferred that most of the compressed portions 7 are formed as linear compressed portions, i.e., thin lines.

Since the compressed portions 7 can be formed in various patterns by heat-embossing the top material layer 1, moreover, they can improve the appearance of the absorbent article.

It should be noted that from the viewpoints of the uniformity in the texture of the top material layer 1, the influence on the stiffness of the entire absorbent article and the appearance, the compressed portions 7 are preferably formed in a generally uniform, regular pattern.

### (Absorbent Body)

The absorbent body 3 can be manufactured from any suitable materials as long as they are water absorbent materials. For example, fluff pulp deposited in a predetermined size and optionally mixed with superabsorbent resin or expanded absorbent material such as hydrophilic urethane foam containing superabsorbent resin or cellulose sponge can be used. For the absorbent body 3, the most preferred is a laminated structure of two or more layers of air-laid pulp, wherein the density is different for different layers so as to increase toward the back material layer 2.

Preferably, the absorbent body 3 is of a size (length and width) almost equal to that of the private parts of women. For example, the length is from 70 to 120 mm and the width W1 is from 20 to 40 mm. If the absorbent body 3 is larger than the private parts (e.g., if the absorbent body 3 extends over the entire length of the absorbent article), a three-dimensional fit in the recesses of the private parts will be impaired to cause a clearance between the absorbent article and the private parts. Therefore, body fluid may possibly leak. In addition, the absorbent body 3 may be deformed on the buttocks that move actively to provide a foreign body sensation.

The top material layer 1 and the back material layer 2 are of the same size, and the absorbent body 3 is rectangular and of a size smaller than that of the layers 1 and 2. The length of the absorbent body 3 is smaller than the length of the top material layer 1 and the back material layer 2; the width W1 is smaller than the minimum width of the top material layer 1 and the back material layer 2 i.e., the minimum distance between the side edges 8 and 8 of the absorbent article. The midpoint between the side edges 6 and 6 of the absorbent body 3 coincides with the midpoint between the side edges 8 and 8 of the absorbent article. In more detail, the side edges 6 and 6 of the absorbent body 3 are inwardly spaced 5 to 15 mm apart from the side edges 8 and 8 of the absorbent article, respectively.

As used herein, the term " central region" refers to a region having the absorbent body 3, while the term " side region" refers to a region located between one side edge 6 of the absorbent body 3 and the corresponding one of the side edges 8 of the absorbent article.

For example, the absorbent body 3 can be formed as follows:

For the absorbent body 3, a single sheet of air-laid pulp having a basis weight of 160 g/m², a thickness of 1.6 mm, a length of 100 mm and a width W1 of 30 mm is treated with hot air for one minute in a 140 °C oven to change the thickness to 2.0 mm. Here, the air-laid pulp that is also called " air-laid nonwoven fabric" comprises 87% by weight of pulp and 13% by weight of PE/PP sheath/core bicomponent synthetic fibers (fineness of 1.7 dtex, length of 13 mm). When heated, the bicomponent synthetic fibers of which the sheath component is formed from PE of a low melting point can function as binder. Alternatively, an adhesive may be employed for bonding the pulp and the bicomponent synthetic fibers.

### (Back Material Layer)

The back material layer 2 can be manufactured from any suitable materials as long as they are liquid-impermeable materials. For example, air-permeable (breathable) plastic film, air-impermeable plastic film or SMS nonwoven fabric formed by stacking and bonding spunbonded nonwoven fabric, meltblown nonwoven fabric and spunbonded nonwoven fabric can be used.

### (Adhesive for Assembly)

When the top material layer 1, the absorbent body 3 and the back material layer 2 are adhered to each other for assembly of the absorbent article or the top material layer 1 or the absorbent body 3 is manufactured by adhering two or more members to each other, an adhesive such as polyolefin hot-melt is used for assembly. The application amount of the adhesive is preferably decreased as much as possible by choosing the kind, application area and application pattern of the adhesive so as to minimize the effect of increasing the stiffness of the absorbent article. At this time, of course, the adhesive has to sufficiently serve as an adhesive for assembly.

### (Pressure Sensitive Adhesive for Preventing Slippage)

On the external surface of back material layer 2, a pressure sensitive adhesive 5 is applied to the whole or partially (e.g., in narrow strips) so as to adhere the absorbent article to a crotch portion of an undergarment such as short panty for preventing slippage during wear. For the pressure sensitive adhesive 5, hydrogenated styrene butadiene rubber, hydrogenated petroleum resin, or a rubber adhesive mixed with paraffin oil or the like is preferably used. This pressure sensitive adhesive 5 is covered with a release paper that is treated with a release agent.

### (Overall Shape and Structure of Absorbent Article)

In the embodiment shown, the absorbent article is of an hourglass shape, wherein the longitudinally opposed end edges that are intended to come into contact with the abdomen and the buttocks of a wearer are outwardly curved and the longitudinally extending side edges 8, 8 are slightly inwardly curved. Preferably, the absorbent article has a maximum length of about 140 mm, a minimum width of about 45 mm near the midpoint between the end edges and a maximum width of about 60 mm near the end edges.

The absorbent article of the present invention preferably has a thickness of 2.0 mm to 20 mm at the central region. If the thickness is less than 2.0 mm, a three-dimensional fit in the recesses of the private parts will be impaired to cause a clearance between the absorbent article and the private parts. Therefore, the effect of preventing leakage of body fluid will be lowered. If the thickness is greater than 20 mm, on the other hand, although the fit can be enhanced to have an effect in preventing leakage of body fluid, a wearer may feel uncomfortable when a pressure is applied on the absorbent article from the wearer's body. More preferably, the thickness is in the range of 2.5 to 5.0 mm. Within this range, a suitable fit can be obtained while effectively preventing the leakage.

In the central region of the absorbent article where the absorbent body 3 is present, moreover, a large number of small liquid passage holes 9 are formed to extend through the top material layer 1 into the absorbent body 3 so that body fluid given to the external surface of the top material layer 1 can be promptly introduced into the absorbent body 3.

The liquid passage holes 9 can be formed by forcing pin-like projections against the top material layer 1 into the absorbent body 3, thereby partially breaking the top material layer 1 and the absorbent body 3 or partially compressing the top material layer 1 and the absorbent body 3. In the sectional view of Fig. 1B, the liquid passage hole 9 has an inner wall portion 9a where the top material layer 1 is present and a bottom portion 9b where the top material layer 1 is not present. It is also possible that the top material layer 1 is present in the bottom portion 9b.

As shown in Figs. 2A and 2B, the liquid passage holes 9 are regularly dotted over the substantially entire central region. However, it should be noted that the liquid passage holes 9 are dotted over a region between compressed portions 7 arranged along one side edge 6 and compressed portions 7 arranged along the other side edge 6, so that all the liquid passage holes 9 are spaced apart from the compressed portions 7 so as not to overlap with the compressed portions 7.

With the liquid passage holes 9 in the central region, body fluid given to the external surface of the top material layer 1 in the central region can flow into the liquid passage holes 9, so that body fluid can be easily absorbed by the absorbent body 3. The compressed portions 7 are formed in regions riding on the side edges 6 i.e., regions of a width W2; and the liquid passage holes 9 are dotted over the central region so as not to overlap with the compressed portions 7. Accordingly, body fluid flowing or diffusing laterally in the top material layer 1 can be blocked by the compressed portions 7 near the side edges of the central region and promptly absorbed by the underlying absorbent body 3 through the liquid passage holes 9.

As has been described hereinabove, the liquid barrier effect of the compressed portions 7 arranged along the side edges of the central region and the liquid passage effect of the liquid passage holes 9 provided in the central region are combined to prevent lateral leakage or exudation of body fluid and further increase absorbency of body fluid.

In the shown embodiments, the compressed portions 7 form a pattern of leaves or the like over the regions having the width W2. This substantially means that liquid barrier zones are formed to extend over the entire length of the top material layer 1 within the regions having the width W2 and riding on the side edges 6. With such liquid barrier zones, body fluid that tends to flow or diffuse from the central region to the side regions can be blocked. In addition, since the ratio of the area occupied by the compressed portions 7 to the area of the liquid barrier zone is not large, the absorbent article can be prevented from being excessively stiffened.

The width W2 of the liquid barrier zone is preferably from 3 mm to 10 mm. If the width W2 is less than 3 mm, the ratio of the area occupied by the compressed portions 7 to the area of the liquid barrier zone will be excessively increased to stiffen the article. If the width W2 is greater than 10 mm, on the other hand, the area for substantial liquid absorption will be excessively decreased within the central region.

Preferably, the width W3 of the region between the liquid barrier zones is at least 0.6 times the width W1 of the absorbent body 3. Within this range, a sufficiently large area can be secured for liquid permeation through the top material layer 1.

### (Manufacturing Method of Absorbent Article)

The absorbent article can be manufactured through processes including material supply, assembly and so on that are ordinarily adopted in the art.

For example, the absorbent article can be manufactured as follows: a single sheet of nonwoven fabric or two or more sheets of nonwoven fabric adhered to each other are treated to restore bulk and then heat-embossed at predetermined regions to obtain a sheet for the top material layer 1. Then, the absorbent body 3 that has been separately manufactured and cut into a predetermined size is adhered to the top material layer 1. The formation of the liquid passage holes 9 can be performed from above the top material layer 1 toward the absorbent body 3 after the absorbent body 3 is adhered to the top material layer 1. Next, the back material layer 2 combined with the pressure sensitive adhesive 5 and the release paper is adhered thereto. Subsequently, the laminate is processed with a pressure roll and a cutter to obtain the absorbent article as final product.

According to the present invention, as has been described hereinabove, since the liquid-permeable top material layer is embossed to have a predetermined pattern of compressed portions along the individual side edges of the absorbent body, body fluid diffusing laterally in the top material layer can be blocked by the compressed portions to prevent lateral leakage or exudation. The body fluid thus blocked can be absorbed by the underlying absorbent body.

Although the present invention has been illustrated and described with respect to exemplary embodiments thereof, it should be understood by those skilled in the art that the foregoing and various other changes, omission and additions may be made therein and thereto, without departing from the spirit and scope of the present invention. Therefore, the present invention should not be understood as limited to the specific embodiments set out above but to include all possible embodiments which can be embodied within a scope encompassed and equivalent thereof with respect to the feature set out in the appended claims.

## Claims

1. An absorbent article comprising:
a liquid-permeable top material layer;
a liquid-impermeable back material layer; and
an absorbent body that is narrower than the top material layer and the back material layer and disposed between the top material layer and the back material layer such that longitudinally extending side edges of the absorbent body are individually inwardly spaced apart from corresponding side edges of the top material layer and the back material layer, wherein
along the individual side edges of the absorbent body, the top material layer only is heat-embossed to have compressed portions, wherein the compressed portions are arranged in a longitudinally spaced relation to each other so that the compressed portions are formed in a substantially continuous, longitudinally extending pattern with regions having no compressed portions left between longitudinally adjacent compressed portions so that at least 80% of each side edge overlaps with the compressed portions, when the top material layer is viewed from a direction perpendicular to each side edge of the absorbent body in a plane substantially flush with the top material layer,
wherein the compressed portions all comprise linear compressed portions, which each extend to surround an uncompressed portion without interruption, and the linear compressed portions form a pattern repeated along the individuals side edges of the absorbent body, the compressed portions being provided in regions riding on the side edges of the absorbent body to leave a non embossed region between the regions having the compressed portions.

2. An absorbent article as set forth in claim 1, wherein the linear compressed portions form a pattern of leaves.

3. An absorbent article as set forth in any preceding claim, wherein liquid passage holes extending through the top material layer into the absorbent body are formed in a region where the absorbent body is present.

4. An absorbent article as set forth in claim 3, wherein the liquid passage holes are distributed within a nonembossed region left between regions having the compressed portions so that all the liquid passage holes are spaced apart from the compressed portions.

## Patentansprüche

1. Absorbierender Artikel, umfassend:
eine flüssigkeitsdurchlässige obere Materialschicht;
eine flüssigkeitsundurchlässige rückseitige Materialschicht; und
einen absorbierenden Körper, der schmaler ist als die obere Materialschicht und die rückseitige Materialschicht und so zwischen der oberen Materialschicht und der rückseitigen Materialschicht angeordnet ist, dass sich in Längsrichtung erstreckende Seitenränder des absorbierenden Körpers von den entsprechenden Seitenrändern der oberen Materialschicht und der rückseitigen Materialschicht jeweils nach innen beabstandet sind, wobei
entlang den jeweiligen Seitenrändern des absorbierenden Körpers nur die obere Materialschicht heißgeprägt ist und daher gestauchte Partien aufweist, wobei die gestauchten Partien in Längsrichtung so voneinander beabstander angeordnet sind, dass die gestauchten Partien in einem im Wesentlichen durchgehenden, sich in Längsrichtung erstreckenden Muster gebildet sind und Bereiche ohne gestauchte Partien zwischen einander in Längsrichtung benachbarten gestauchten Partien verbleiben, so dass mindestens 80% eines jeden Seitenrandes mit den gestauchten Partien überlappen, wenn die obere Materialschicht aus einer Richtung betrachtet wird, die senkrecht zu dem jeweiligen Seitenrand des absorbierenden Körpers in einer im Wesentlichen mit der oberen Materialschicht fluchtenden Ebene verläuft,
wobei die gestauchten Partien jeweils lineare gestauchte Partien aufweisen, die sich jeweils ohne Unterbrechung rund um eine nicht gestauchte Partie erstrecken, und wobei die linearen gestauchten Partien ein sich entlang den jeweiligen Seitenrändern des absorbierenden Körpers wiederholendes Muster bilden, wobei die gestauchten Partien in Bereichen vorgesehen sind, die an den Seitenrändern des absorbierenden Körpers sitzen, und zwischen den Bereichen mit den gestauchten Partien ein nicht geprägter Bereich verbleibt.

2. Absorbierender Artikel nach Anspruch 1, wobei die linearen gestauchten Partien ein Blattmuster bilden.

3. Absorbierender Artikel nach einem der vorhergehenden Ansprüchs, wobei sich durch die obere Materialschicht in den absorbierenden Körper erstreckende Flüssigkeitsdurchgangslöcher in einem Bereich gebildet sind, wo der absorbierende Körper vorhanden ist.

4. Absorbierender Artikel nach Anspruch 3, wobei die Flüssigkeitsdurchgangslöcher innerhalb eines nicht geprägten Bereichs verteilt sind, der zwischen Bereichen mit den gestauchten Partien verbleibt, so dass alle Flüssigkeitsdurchgangslöcher von den gestauchten Partien beabstandet sind.

## Revendications

1. Article absorbant comportant :
une couche textile supérieure perméable aux liquides ;
une couche textile de support imperméable aux liquides ; et
un corps absorbant qui est plus étroit par rapport à la couche textile supérieure et à la couche textile de support et disposé entre la couche textile supérieure et la couche textile de support de sorte que des bords latéraux s'étendant dans le sens longitudinal du corps absorbant sont espacés l'un de l'autre individuellement vers l'intérieur par rapport aux bords latéraux correspondants de la couche textile supérieure et de la couche textile de support, dans lequel
le long des bords latéraux individuels du corps absorbant, seule la couche textile supérieure est gaufrée à chaud pour obtenir des parties comprimées, dans lequel les parties comprimées sont arrangées en une relation espacée dans le sens longitudinal les unes par rapport aux autres de sorte que les parties comprimées sont formées selon un motif s'étendant dans le sens longitudinal et sensiblement continu avec des régions n'ayant aucune partie comprimée laissée entre des parties comprimées adjacentes dans le sens longitudinal de sorte qu'au moins 80 % de chaque bord latéral chevauche les parties comprimées, quand la couche textile supérieure est vue depuis un sens perpendiculaire à chaque bord latéral du corps absorbant dans un plan se trouvant sensiblement au même niveau que la couche textile supérieure,
dans lequel les parties comprimées comportent toutes des parties comprimées linéaires, qui s'étendent chacune pour entourer une partie non comprimée sans interruption, et les parties comprimées linéaires forment un motif répété le long des bords latéraux individuels du corps absorbant, les parties comprimées étant mises en oeuvre dans des régions montant sur les bords latéraux du corps absorbant pour laisser une région non gaufrée entre les régions ayant les parties comprimées.

2. Article absorbant selon la revendication 1, dans lequel les parties comprimées linéaires forment un motif de feuilles.

3. Article absorbant selon l'une quelconque des revendications précédentes, dans lequel des trous de passage pour liquide s'étendant au travers de la couche textile supérieure jusque dans le corps absorbant sont formés dans une région où le corps absorbant est présent.

4. Article absorbant selon la revendication 3, dans lequel les trous de passage pour liquide sont répartis dans les limites d'une région non gaufrée laissée entre des régions ayant les parties comprimées de sorte que tous les trous de passage pour liquide sont espacés à distance des parties comprimées.
